# EUROPEAN PATENT APPLICATION

(11) **EP 3 718 523 A1**
(43) Date of publication of application: **07.10.2020**
(21) Application number: 18883573.0
(22) Date of filing: 27.11.2018
(51) Int. Cl.: A61F 13/47, A61F 13/15, A61F 13/511, A61F 13/53, A61F 13/534, A61F 13/536

(54) **ABSORBENT ARTICLE PACKAGING AND PRODUCTION METHOD FOR ABSORBENT ARTICLE PACKAGING**

(30) Priority: 28.11.2017 JP 2017228428
(71) Applicant: Kao Corporation, Chuo-ku Tokyo 103-8210 (JP)
(72) Inventor: MATSUNAGA, Ryuji, Haga-gun Tochigi 321-3497 (JP); HARADA, Takuaki, Haga-gun Tochigi 321-3497 (JP); MOTEGI, Tomoyuki, Haga-gun Tochigi 321-3497 (JP); KATO, Yuki, Haga-gun Tochigi 321-3497 (JP); IWASA, Hiroyuki, Haga-gun Tochigi 321-3497 (JP)
(74) Representative: Vossius & Partner Patentanwälte Rechtsanwälte mbB
(86) International application number: PCT/JP2018/043488
(87) International publication number: WO 2019/107327

(57) **Abstract**

Disclosed is an individual package (100) of a napkin (101) having a longitudinal direction (xl) corresponding to the wearer's front-rear direction and a lateral direction (yl) orthogonal to the longitudinal direction, the napkin (101) including a topsheet (102), a backsheet (103), and an absorbent core (110) arranged between the two sheets (102, 103), the napkin being packaged in a folded-up state. The napkin (101) is folded up in the longitudinal direction (xl) of the napkin (101), with the topsheet (102) on the inside, along a first folded/bent portion (IP1) and a second folded/bent portion (IP2) that extend in the lateral direction (yl) of the napkin (101). The absorbent core (110) includes a plurality of sheet fragments (10bh) including synthetic fibers (10b), and the sheet fragments (lObh) are provided at least on the topsheet (102) side in a thickness direction of the absorbent core (110).

## Description

### Technical Field

The present invention relates to packages of absorbent articles, such as disposable diapers and sanitary napkins, and methods for manufacturing such packages.

### Background Art

There are absorbent articles, such as disposable diapers, sanitary napkins and incontinence pads, that are packaged by a packaging material in a folded-up state. Packaging an absorbent article in a folded-up state, however, has a drawback in that creases are likely to be formed in sections where the article was folded when the packaged state is released.

Applicant has previously proposed an individual package of an absorbent article, wherein the absorbent article includes: an annular leakage-preventing groove that extends in the longitudinal direction and integrates the topsheet and the absorbent member; and central grooves that extend in the lateral direction between left and right grooves constituting the annular leakage-preventing groove. A fold line for the individual package is provided at a specific position with respect to the annular leakage-preventing groove and each central groove. In this way, when the individual package is opened from its packaged state, creases are less likely to be formed in sections of the topsheet that correspond to sections where the absorbent article was folded (Patent Literature 1).

### Citation List

### Patent Literature

Patent Literature 1: JP 2010-178932A

### Summary of Invention

The present invention relates to an absorbent article package that includes an absorbent article including a topsheet, a backsheet, and an absorbent core arranged between the topsheet and the backsheet, the absorbent article having a longitudinal direction corresponding to a front-rear direction of a wearer and a lateral direction orthogonal to the longitudinal direction, the absorbent article being packaged in a folded-up state. The absorbent article is folded up, with the topsheet on the inside, along a folded/bent portion that extends in the lateral direction of the absorbent article. The absorbent core includes a plurality of sheet fragments including synthetic fibers; and the sheet fragments are provided at least on the topsheet side in a thickness direction of the absorbent core.

The present invention also relates to a method for manufacturing an absorbent article package that includes an absorbent article including a topsheet, a backsheet, and an absorbent core arranged between the topsheet and the backsheet, the absorbent article having a longitudinal direction corresponding to a front-rear direction of a wearer and a lateral direction orthogonal to the longitudinal direction, the absorbent article being packaged in a folded-up state. The invention involves a core forming step of forming the absorbent core by accumulating a plurality of sheet fragments including synthetic fibers. The invention involves an article forming step of first forming a continuous absorbent article strip by superposing, on one another, the absorbent core and a continuous topsheet that is being transported, and then cutting the continuous absorbent article strip, to form the absorbent article. The invention involves a fold-up step of folding the absorbent article, with the topsheet on the inside, so as to form a folded/bent portion that extends in the lateral direction of the absorbent article. In the fold-up step, the folding portion is formed by performing folding at a section where the sheet fragments are present in the absorbent core.

### Brief Description of Drawings

[Fig. 1] Fig. 1 is a perspective view schematically illustrating an individual package of a sanitary napkin which is a preferred embodiment of an absorbent article package of the present invention.
[Fig. 2] Fig. 2 is a perspective view schematically illustrating a state in which the individual package illustrated in Fig. 1 has been opened by removing a fastening tape.
[Fig. 3] Fig. 3 is a plan view, as viewed from the skin-facing surface side (topsheet side) of the sanitary napkin, schematically illustrating a spread-open state of the individual package illustrated in Fig. 2.
[Fig. 4] Fig. 4 is a lateral cross-sectional view schematically illustrating a cross section along line IV-IV of the individual package illustrated in Fig. 3.
[Fig. 5] Fig. 5 is a schematic side view schematically illustrating a preferred embodiment of a manufacturing device for manufacturing the individual package illustrated in Fig. 1.
[Fig. 6] Fig. 6 is a perspective view illustrating an absorbent member forming portion of the manufacturing device illustrated in Fig. 5.
[Fig. 7] Fig. 7 is a partially enlarged side view of a supplying portion in a core forming portion illustrated in Fig. 6.
[Fig. 8] Fig. 8 is a schematic plan view of an individual package forming portion of the manufacturing device illustrated in Fig. 5.
[Fig. 9] Fig. 9 is a side view schematically illustrating another embodiment of a core forming portion of the manufacturing device illustrated in Fig. 5.

### Description of Embodiments

The method of Patent Literature 1, wherein folding creases are suppressed by the arrangement of the leakage-preventing groove, reduces the degree of freedom in the design of the leakage-preventing groove, thus causing difficulty in achieving both leakage preventability and suppression of creases. On the other hand, Inventors have found that the reason that creases are likely to be formed in the topsheet when the packaged state is released is because pulp fibers, which are hydrophilic fibers constituting the absorbent member, get deformed when the absorbent article is made into a packaged state, and it is difficult to return the deformed pulp fibers to their original shape even when the packaged state is released. Patent Literature 1 neither describes nor suggests anything about the use of nonwoven fabric fragments as a constituent material for the absorbent member in order to prevent the formation of creases in the topsheet when releasing the packaged state.

The present invention thus relates to an absorbent article package in which folding creases are less likely to be formed in the topsheet. The present invention also relates to a method for manufacturing an absorbent article package in which folding creases are less likely to be formed in the topsheet.

The present invention is described below according to preferred embodiments thereof with reference to the drawings. An absorbent article package of the present invention is used for absorbing and retaining body fluid excreted from the body, with examples mainly including urine and menstrual blood. Examples of absorbent articles include disposable diapers, sanitary napkins, incontinence pads, and pantiliners, but are not limited thereto, and widely encompass articles used for absorbing liquids discharged from the human body. The "absorbent article package" encompasses, for example, a package containing a plurality of folded-up disposable diapers, and an individual package in which a single sanitary napkin etc. has been packaged separately. Hereinbelow, the absorbent article package of the present invention is described by employing an example of an individual package 100 of a sanitary napkin 101 (referred to hereinafter also as "napkin 101") which is a preferred embodiment of the absorbent article package.

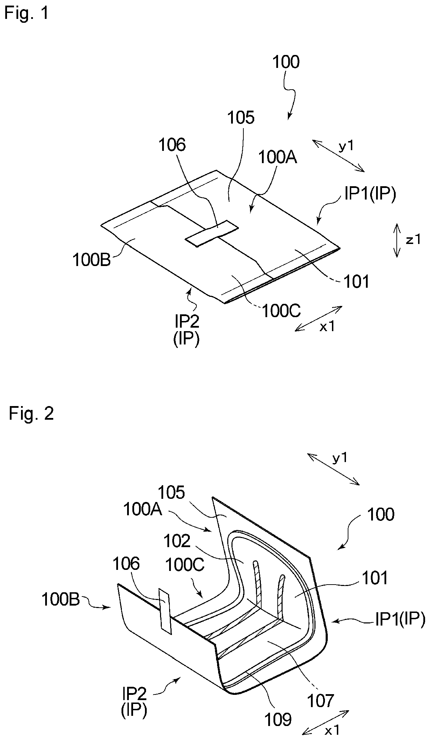

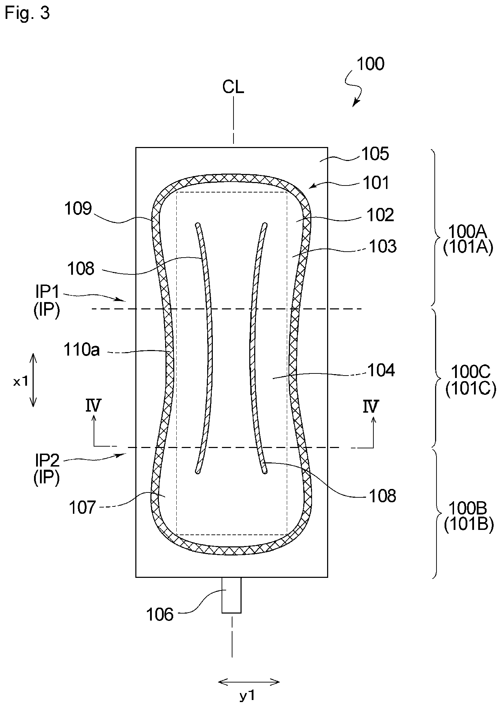

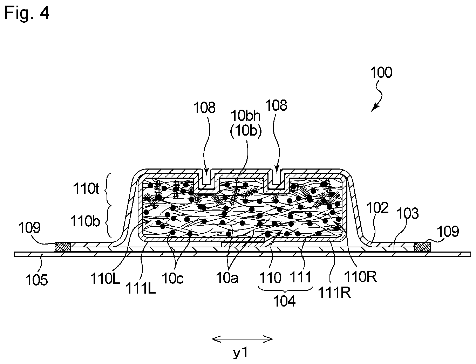

Fig. 1 illustrates a perspective view of an individual package 100 in an unopened state. Fig. 2 is a perspective view illustrating a state in which a fastening tape 106 of the individual package 100 illustrated in Fig. 1 has been detached and opened. Fig. 3 illustrates a plan view of a topsheet 102 side of the individual package 100 of Fig. 2 in a spread-open state. Fig. 4 illustrates a lateral cross-sectional view, taken along line IV-IV, of the individual package 100 in the spread-open state illustrated in Fig. 1.

As illustrated in Fig. 1, the individual package 100 includes: a longitudinal direction x1 corresponding to the front-rear direction of a wearer; and a lateral direction y1 orthogonal to the longitudinal direction. As illustrated in Figs. 3 and 4, the individual package 100 includes: a napkin 101 that includes a liquid-permeable topsheet 102; a sparingly liquid-permeable backsheet 103; and a liquid-retentive absorbent member 104; and a packaging material 105 in which the napkin 101 is packaged. In the individual package 100, the packaging material 105 is peelably attached to the non-skin-facing surface of the napkin 101 by means of an adhesive portion 107.

In an unopened state, the napkin 101 is folded up, with the topsheet 102 on the inside, along folded/bent portions IP that extend along the lateral direction y1. As illustrated in Fig. 1, the folded/bent portions IP include a first folded/bent portion IP1 and a second folded/bent portion IP2 that extend in the lateral direction y1 and are separated from one another in the longitudinal direction x1 of the napkin 101. In its spread-open state, the individual package 100 is divided into: a central region 100C located between the first folded/bent portion IP1 and the second folded/bent portion IP2; and a first folded region 100A and a second folded region 100B which extend outward in the longitudinal direction x1 respectively from the first folded/bent portion IP1 and the second folded/bent portion IP2. In the individual package 100 in its unopened state, the first folded region 100A and the second folded region 100B are place on top of the central region 100C in this order in the thickness direction Z; thus, the central region 100C constitutes a lower layer, the second folded region 100B constitutes an upper layer, and the first folded region 100A constitutes an intermediate layer arranged between the two layers 100C, 100B. In its unopened state, the individual package 100 is sealed by a fastening tape 106.

As illustrated in Fig. 3, the napkin 101 and the packaging material 105 have a shape that is long in the longitudinal direction x1 when the individual package 100 is spread open, and the length direction matches the longitudinal direction x1, whereas the width direction orthogonal to the length direction matches the lateral direction y1. The longitudinal direction x1 is also the direction extending from the wearer's front side toward the rear side via the crotch portion. In this description, the skin-facing surface is the surface of the napkin 101, as well as its constituent members (e.g., the absorbent member 104), that faces the wearer's skin side in a worn state (i.e., the side relatively closer to the wearer's skin), whereas the non-skin-facing surface is the surface of the napkin 101, as well as its constituent members, that faces the opposite side from the wearer's skin side in a worn state (i.e., the side relatively farther from the wearer's skin). Herein, "worn state" refers to a state in which the absorbent article is maintained in its ordinary, proper wearing/attachment position (i.e., the correct wearing/attachment position of the absorbent article), and does not include cases where the absorbent article is deviated from the aforementioned wearing/attachment position. In the individual package 100 of the napkin 101, the thickness direction is described as the z direction.

As illustrated in Figs. 1 and 2, the packaging material 105 packages the entire napkin 101. As illustrated in Fig. 3, the area of the packaging material 105 in a planar view is larger than the area of the napkin 101 in a planar view. As illustrated in Fig. 3, the packaging material 105 has a rectangular shape in a planar view, and its length direction matches the longitudinal direction x1 of the napkin 101. The napkin 101 is fixed on the inner side of the packaging material 105 by means of an adhesive portion 107 provided on the non-skin-facing surface of the napkin 101. The adhesive portion 107 also functions as a displacement prevention portion that fixes the napkin 101 to the clothing when the napkin 101 is worn. The arrangement pattern of the adhesive portion 107 can be designed as appropriate with consideration given, for example, to the displacement prevention function. The packaging material 105 packages the entire napkin 101 by being folded up along the folded/bent portions IP together with the napkin 101. For the packaging material 105, it is possible to use, without particular limitation, any material ordinarily employed for absorbent articles such as pantiliners and sanitary napkins. For the packaging material 105, it is possible to use, for example, a film or a nonwoven fabric.

As illustrated in Figs. 3 and 4, the napkin 101 includes a topsheet 102 to be arranged on the wearer's skin side, a backsheet 103 to be arranged on the wearer's non-skin side, and an absorbent member 104 arranged between the two sheets 102, 103. As described further below, the absorbent member 104 includes an absorbent core 110 and a liquid-permeable core-wrap sheet 111 covering the absorbent core 110. It can thus be restated that the napkin 101 includes a topsheet 102, a backsheet 103, and an absorbent core 110 arranged between the two sheets 102, 103. As illustrated in Fig. 3, the napkin 101 includes: a front region 101A to be arranged on the wearer's front side; a rear region 101B to be arranged on the rear side; and an excretion section region 101C located between the two regions. As illustrated in Fig. 3, the excretion section region 101C matches the central region 100C of the individual package 100. Stated differently, the excretion section region 101C is a section sandwiched between the first folded/bent portion IP1 and the second folded/bent portion IP2. The front region 101A corresponds to the first folded region 100A of the individual package 100, and the rear region 101B corresponds to the second folded region 100B of the individual package 100.

Although the planar-view shape of the absorbent article constituting the package of the present invention is not particularly limited, the napkin 101 is formed so as to be long in the longitudinal direction x1 and have left-right symmetry with respect to a center line CL extending in the longitudinal direction x1, as illustrated in Fig. 3. The longitudinal direction x1 is also a direction parallel to the center line CL. The napkin 101 is shaped such that, in the excretion section region 101C (i.e., central area in the longitudinal direction x1), both lateral sides along the longitudinal direction x1 are narrowed inwardly in the lateral direction y1.

The absorbent core 110 includes a depression 108 that is depressed from the topsheet 102 side toward the backsheet 103 side. More specifically, as illustrated in Figs. 3 and 4, the napkin 101 includes a depression 108 formed in a manner that the topsheet 102 and the absorbent member 104 are integrally depressed toward the backsheet 103 side. Stated differently, the depression 108 is formed such that the topsheet 102, the absorbent core 110, and the core-wrap sheet 111 arranged therebetween are depressed integrally. The depression 108 is formed by compressing the napkin 101 from its skin-facing surface side, i.e., the topsheet 102 side, toward the backsheet 103 side. Examples of compression include known embossing processes, such as embossing involving heat or ultrasonic embossing. Due to the method for forming the depression 108, the topsheet 102 and the absorbent member 104 may be integrated by thermal fusion-bonding at the bottom portion of the depression 108.

In the absorbent article of the present invention, the planar-view shape and arrangement pattern of the depression 108 are not limited to the linearly-extending planar-view shape as illustrated in Fig. 3, and may be, for example, constituted by individually separate dots having any shape, such as circular, elliptic, rectangular, triangular, star-shaped, or heart-shaped. The depth of the depression 108 does not necessarily have to be constant over the entire length in the length direction, but may be partially different, for example. The shape, arrangement, etc. of the linear depression 108 are not limited to the illustrated configuration, and may be designed like a so-called leakage-preventing groove in this type of absorbent article; the planar-view shape may include straight and/or curved lines, wherein each line may be a continuous line or a broken line.

As illustrated in Fig. 3, the topsheet 102 of the napkin 101 covers the entire region of the skin-facing surface of the absorbent member 104. The backsheet 103 covers the entire region of the non-skin-facing surface of the absorbent member 104. The respective outer edges 109 of the topsheet 102 and the backsheet 103 are joined together by a known joining means, such as an adhesive, heat sealing, or ultrasonic sealing. Each of the topsheet 102 and the backsheet 103 is joined to the absorbent member 104 by an adhesive. For the topsheet 102 and the backsheet 103, it is possible to use, without particular limitation, various types of materials conventionally used in absorbent articles such as sanitary napkins. For example, for the topsheet 102, it is possible to use a nonwoven fabric with a single-layer or multi-layer structure, or a porous film. For the backsheet 103, it is possible to use, for example, a moisture-permeable resin film.

As illustrated in Fig. 3, the absorbent member 104 has a shape that is long in the longitudinal direction x1, which corresponds to the wearer's front-rear direction, when the napkin 101 is worn. The absorbent member's length direction matches the longitudinal direction x1, and the width direction orthogonal to the length direction matches the lateral direction y1. The absorbent member 104 includes an absorbent core 110 and a liquid-permeable core-wrap sheet 111 covering the absorbent core 110. The core-wrap sheet 111 is a single continuous sheet having a width that is from 2 to 3 times the length, in the lateral direction y1, of the absorbent core 110. As illustrated in Fig. 4, the core-wrap sheet 111 covers the entire region of the skin-facing surface of the absorbent core 110, and also extends outward in the lateral direction y1 from the absorbent core 110's respective lateral side edges 110R, 110L which extend along the longitudinal direction x1. The core-wrap sheet's extension portions 111R, 111L are each wrapped downward under the absorbent core 110 and thereby cover the entire region of the non-skin-facing surface of the absorbent core 110. It should be noted that, in the present invention, the core-wrap sheet does not have to be a single sheet, and may, for example, include two sheets, i.e., a single skin-side core-wrap sheet covering the skin-facing surface of the absorbent core 110, and a single non-skin-side core-wrap sheet separate from the skin-side core-wrap sheet and covering the non-skin-facing surface of the absorbent core 110.

As illustrated in Fig. 4, the absorbent core 110 includes the aforementioned depression 108. More specifically, a section on the topsheet 102 side corresponding to the depression 108 is concavely depressed integrally with the topsheet 102 from the topsheet 102 side toward the backsheet 103 side. By concavely depressing the absorbent core 110 by the depression 108, twisting/bunching can be suppressed when the napkin is worn. Also, the absorbent core 110 is highly densified by pressing-in the depression 108 deeply, which can improve liquid capturability.

As illustrated in Fig. 4, the absorbent core 110 includes a plurality of sheet fragments 10bh including synthetic fibers 10b (simply referred to hereinafter also as "sheet fragments 10bh"). The sheet fragments 10bh are provided at least on the topsheet 102 side. The sheet fragments 10bh have a fixed size whose shape and dimensions are substantially uniform.

As illustrated in Fig. 4, each sheet fragment 10bh has a substantially rectangular shape. The average length of the sheet fragments 10bh is preferably from 0.3 to 30 mm, more preferably from 1 to 15 mm, even more preferably from 2 to 10 mm. Herein, in cases where each sheet fragment 10bh is a rectangle, the average length refers to the average value of the length of a side in the length direction. In cases where each sheet fragment 10bh is a square, the average length refers to the average value of the length of any one of the four sides. When the average length of the sheet fragments 10bh is 0.3 mm or greater, a sparse structure can easily be formed in the absorbent member 104. When the average length is 30 mm or less, the absorbent member 104 is less likely to cause an unnatural feel to the wearer, and absorbency is less likely to become uneven depending on the positions within the absorbent member 104. The average width of the sheet fragments 10bh is preferably from 0.1 to 10 mm, more preferably from 0.3 to 6 mm, even more preferably from 0.5 to 5 mm. Herein, in cases where each sheet fragment 10bh is a rectangle, the average width refers to the average value of the length of a side in the shorter direction. In cases where each sheet fragment 10bh is a square, the average width refers to the average value of the length of any one of the four sides. When the average width of the sheet fragments 10bh is 0.1 mm or greater, a sparse structure can easily be formed in the absorbent member 104. When the average width is 10 mm or less, the absorbent member 104 is less likely to cause an unnatural feel to the wearer, and absorbency is less likely to become uneven depending on the positions within the absorbent member 104. The average thickness of the sheet fragments 10bh is preferably from 0.001 to 10 mm, more preferably from 0.01 to 5 mm.

Other than the sheet fragments 10bh, the absorbent core 110 also includes hydrophilic fibers 10a. For the fiber materials forming the absorbent core 110, various materials conventionally used in absorbent cores 110 for absorbent articles can be used without particular limitation. Examples of the hydrophilic fibers 10a include pulp fibers, rayon fibers, and cotton fibers. Examples of the synthetic fibers 10b include short fibers made of polyethylene, polypropylene, or polyethylene terephthalate. The sheet fragments 10bh are not particularly limited so long as they are in a sheet form, but are preferably a nonwoven fabric.

The absorbent core 110 does not need to include the hydrophilic fibers 10a so long as it includes the sheet fragments 10bh. In cases where the hydrophilic fibers 10a are included, the content mass ratio between the sheet fragments 10bh and the hydrophilic fibers 10a (i.e., the content mass of the sheet fragments 10bh to the content mass of the hydrophilic fibers 10a) in the absorbent core 110 is not particularly limited, and may be adjusted as appropriate depending on the type of the sheet fragments 10bh and the hydrophilic fibers 10a. For example, in cases where the synthetic fibers included in the sheet fragments 10bh is PET/polyethylene and the hydrophilic fibers 10a is cellulose, it is preferable that, from the viewpoint of suppressing the formation of folding creases in the napkin 101 after opening an unopened individual package 100, the content mass ratio between the sheet fragments 10bh and the hydrophilic fibers 10a is preferably 0.01 or greater, more preferably 0.1 or greater, and preferably 100 or less, more preferably 10 or less, and preferably from 0.01 to 100, more preferably from 0.1 to 10.

The content of the sheet fragments 10bh in the absorbent core 110 with respect to the entire mass of the absorbent core 110 in a dry state is preferably 1 mass% or greater, more preferably 10 mass% or greater, and preferably 100 mass% or less, more preferably 90 mass% or less, and preferably from 1 to 100 mass%, more preferably from 10 to 90 mass%.

The content of the hydrophilic fibers 10a in the absorbent core 110 with respect to the entire mass of the absorbent core 110 in a dry state is preferably 1 mass% or greater, more preferably 10 mass% or greater, and preferably 99 mass% or less, more preferably 90 mass% or less, and preferably from 1 to 99 mass%, more preferably from 10 to 90 mass%.

The basis weight of the sheet fragments 10bh in the absorbent core 110 is preferably 1 g/m² or greater, more preferably 20 g/m² or greater, and preferably 1000 g/m² or less, more preferably 800 g/m² or less, and preferably from 1 to 1000 g/m², more preferably from 20 to 800 g/m².

The basis weight of the hydrophilic fibers 10a in the absorbent core 110 is preferably 1 g/m² or greater, more preferably 20 g/m² or greater, and preferably 1000 g/m² or less, more preferably 800 g/m² or less, and preferably from 1 to 1000 g/m², more preferably from 20 to 800 g/m².

In addition to the sheet fragments 10bh and the hydrophilic fibers 10a, the absorbent core 110 includes absorbent particles 10c. Examples of the absorbent particles 10c include starch-based, cellulose-based, synthetic polymer-based, and superabsorbent polymer-based particles. Examples of superabsorbent polymers that may be used include starch-acrylic acid (acrylate) graft copolymers, saponified products of starch-acrylonitrile copolymers, crosslinked products of sodium carboxymethyl cellulose, and acrylic acid (acrylate) polymers.

The content of the absorbent particles 10c in the absorbent core 110 with respect to the entire mass of the absorbent core 110 in a dry state is preferably 0 mass% or greater, more preferably 1 mass% or greater, and preferably 90 mass% or less, more preferably 70 mass% or less, and preferably from 0 to 90 mass%, more preferably from 1 to 70 mass%.

The basis weight of the absorbent particles 10c in the absorbent core 110 is preferably 0 g/m² or greater, more preferably 5 g/m² or greater, and preferably 1000 g/m² or less, more preferably 800 g/m² or less, and preferably from 0 to 1000 g/m², more preferably from 5 to 800 g/m².

For constituent members constituting the absorbent core 110, it is also possible to use, for example, deodorants and antibacterial agents as necessary. Examples of the core-wrap sheet 111 include fiber sheets, such as tissue paper and liquid-permeable nonwoven fabrics.

As illustrated in Fig. 4, the absorbent core 110 includes, in the thickness direction Z: a first layer 110t including fiber materials in which the sheet fragments 10bh and the hydrophilic fibers 10a are mixed, and the absorbent particles 10c; and a second layer 110b including the fiber materials in which the density of presence of the sheet fragments 10bh is smaller than in the first layer 110t, and the absorbent particles 10c. Herein, the density of presence of the sheet fragments 10bh refers to the number of sheet fragments 10bh that are present per 1 cm² in a discretionary cross section that is parallel to the thickness direction Z of the absorbent core 110. In the present embodiment, the first layer 110t is arranged on the topsheet 102 side, and the second layer 110b is arranged on the backsheet 103 side. In the first layer 110t of the absorbent core 110, the sheet fragments 10bh and the hydrophilic fibers 10a are entangled with one another. Stated differently, in the first layer 110t, the hydrophilic fibers are entangled and bound with the sheet fragments 10bh. On the other hand, there is no sheet fragment 10bh present in the second layer 110b of the absorbent core 110 of the present embodiment, and the second layer is constituted only by the hydrophilic fibers 10a and the absorbent particles 10c, wherein the hydrophilic fibers 10a are entangled and bound with one another. According to the individual package 100 formed by folding up the napkin 101 which includes the absorbent core 110 including the sheet fragments 10bh, the sheet fragments 10bh having recoverability that allows easy recovery to the original state are arranged on the topsheet 102 side of the absorbent core 110-i.e., the valley-fold side. Thus, when the napkin 101 is spread-open from its individually-packaged state, folding creases are less likely to be formed on the surface of the topsheet 102 corresponding to the folded/bent portions IP in the napkin 101. Particularly, in the present napkin 101, the sheet fragments 10bh are arranged only in the first layer 110t on the topsheet 102 side of the absorbent core 110, and the sheet fragments 10bh are not present on the backsheet 103 side of the absorbent core 110-i.e., not present in the second layer 110b on the mountain-fold side. Thus, the hydrophilic fibers 10a in the second layer 110b can maintain the folded shape, whereas when the napkin 101 is spread open, the sheet fragments 10bh in the first layer 110t can suppress the formation of creases. Further, since the formation of creases can be suppressed by this arrangement of the sheet fragments 10bh, it becomes possible to freely design the depression 108 formed by compression.

The synthetic fibers 10b included in the absorbent core 110 are not included in a defibrated state where they are separated into single, individual fibers, but are instead included as sheet fragments 10bh having an intended size. This achieves excellent recoverability allowing easy recovery to the original state, and thus folding creases can be suppressed effectively. Further, dispersing the sheet fragments 10bh, which have an intended size, will make it less likely to cause uncomfortableness of contacting a foreign object while using the napkin 101, and can also achieve high-speed absorption of body fluid.

From the viewpoint of suppressing the formation of folding creases in the napkin 101 after opening an unopened individual package 100, the density of presence of the sheet fragments 10bh in the first layer 110t of the absorbent core 110 is preferably 1 piece/cm² or greater, more preferably 5 pieces/cm² or greater, and preferably 500 pieces/cm² or less, more preferably 200 pieces/cm² or less, and preferably from 1 to 500 pieces/cm², more preferably from 5 to 200 pieces/cm².

From the same viewpoint, the density of presence of the sheet fragments 10bh in the second layer 110b of the absorbent core 110 is preferably 0 pieces/cm² or greater, more preferably 1 piece/cm² or greater, and preferably 500 pieces/cm² or less, more preferably 200 pieces/cm² or less, and preferably from 0 to 500 pieces/cm², more preferably from 20 to 200 pieces/cm².

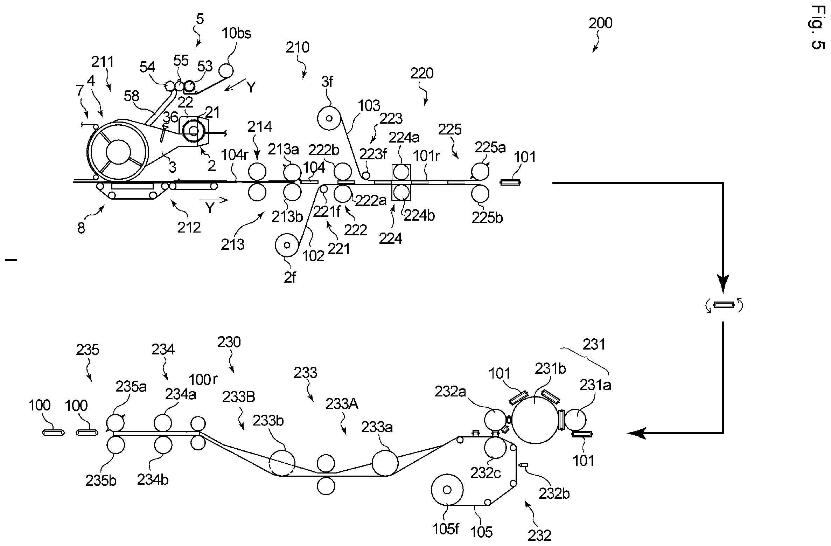

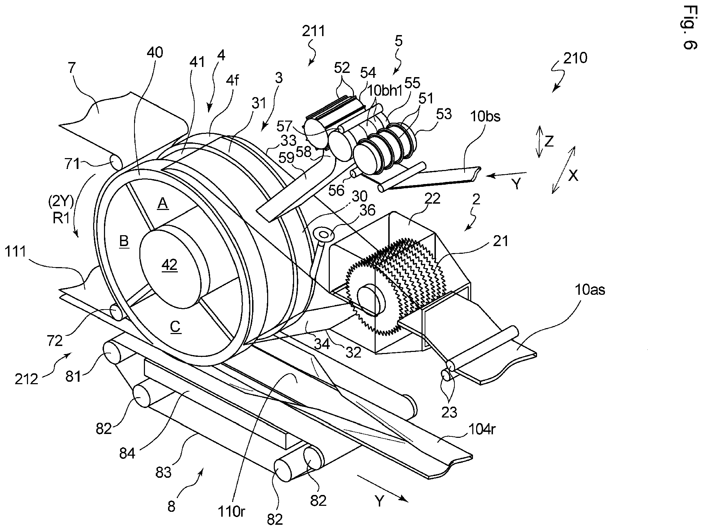

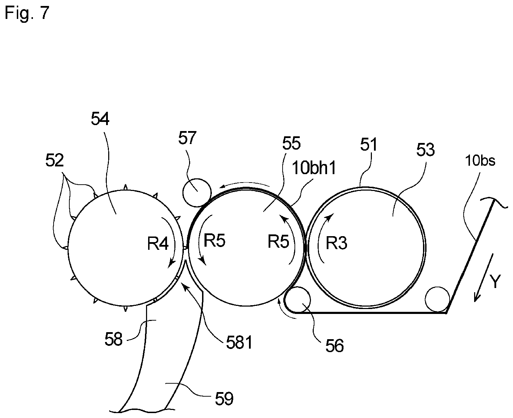

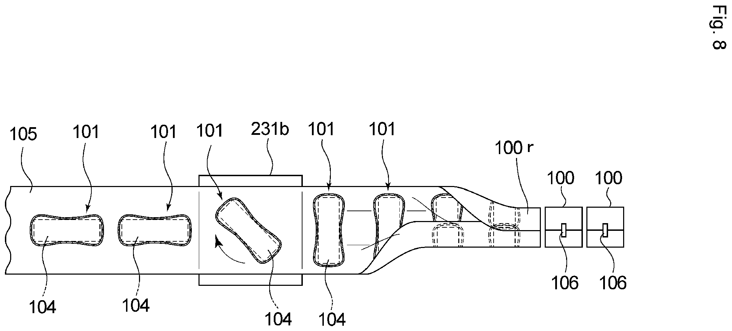

Next, an absorbent article package manufacturing method according to an embodiment of the present invention is described with reference to Figs. 5 to 8, taking, as an example, a method for manufacturing an individual package 100 of a napkin 101. Fig. 5 illustrates an overall configuration of an embodiment of a manufacturing device 200 used for performing the manufacturing method of the embodiment. Fig. 6 illustrates a perspective view of an absorbent member forming portion 210 of the manufacturing device 200. Fig. 7 illustrates a partially enlarged side view of a supplying portion 5 in the absorbent member forming portion 210 for supplying sheet fragments 10bh. Fig. 8 illustrates a schematic plan view of an individual package forming portion 230 of the manufacturing device. On describing the method for manufacturing the individual package 100, first, the manufacturing device 200 will be described.

As illustrated in Fig. 5, the manufacturing device 200 includes, from the upstream side toward the downstream side in the transporting direction: an absorbent member forming portion 210 for forming absorbent members 104; an absorbent article forming portion 220 for forming napkins 101; and an individual package forming portion 230 for forming individual packages 100.

In the description below, the direction in which a continuous absorbent member strip 104r and a continuous synthetic fiber sheet lObs including the synthetic fibers 10b are transported is the Y direction, the width direction of the synthetic fiber sheet lObs and the continuous absorbent member strip 104r being transported and the direction orthogonal to the transporting direction are the X direction, and the thickness direction of the synthetic fiber sheet lObs and the continuous absorbent member strip 104r being transported is the Z direction. Further, the later-described first direction is a direction extending in the transporting direction Y, and refers to a direction wherein the angle formed between it and the transporting direction Y is within a range of less than 45 degrees. In the present embodiment, the first direction matches the direction parallel to the transporting direction Y Further, the later-described second direction is a direction intersecting with the first direction. In the present embodiment, the second direction is a direction orthogonal to the first direction, and matches the direction parallel to the width direction of the synthetic fiber sheet lObs and the absorbent member 104 being transported.

As illustrated in Fig. 5, the absorbent member forming portion 210 includes: a core forming portion 211 for forming absorbent cores 110; a covering portion 212 for covering the absorbent cores 110 with a core-wrap sheet 111 to form a continuous absorbent member strip 104r; a pressing portion 214 for compressing the continuous absorbent member strip 104r in the thickness direction Z; and an absorbent member cutting portion 213 for cutting the continuous absorbent member strip 104r to form individual absorbent members 104.

As illustrated in Figs. 5 and 6, the core forming portion 211 includes: a defibrating portion 2 that defibrates a hydrophilic sheet 10as including the hydrophilic fibers 10a; a duct 3 that transports the material of the absorbent member 104 by carrying it on an airflow; a supplying portion 5 that supplies the sheet fragments 10bh in midstream of the duct 3; a rotary drum 4 that is arranged downstream of the duct 3 adjacent thereto and that includes an accumulating portion in which the material of the absorbent member 104 is accumulated; and a press-down belt 7 arranged along the rotary drum 4's outer circumferential surface 4f located on the opposite side from the duct 3.

As illustrated in Fig. 6, the defibrating portion 2 includes: a defibrating machine 21 that defibrates the hydrophilic sheet 10as; and a casing 22 that covers the upper side of the defibrating machine 21. The defibrating portion 2 is a section that supplies, to inside the duct 3, the defibrated hydrophilic fibers 10a which are a material of the absorbent member 104. The defibrating portion 2 also includes a pair of feed rollers 23, 23 that supplies the hydrophilic sheet 10as to the defibrating machine 21.

As illustrated in Fig. 6, the duct 3 extends from the defibrating portion 2 up to the rotary drum 4, and the duct 3's opening on the downstream side covers the outer circumferential surface 4f which is located at the rotary drum 4's space A which is maintained at a negative pressure. The duct 3 includes a top plate 31 forming the top surface, a bottom plate 32 forming the bottom surface, and side walls 33, 34 forming the respective side surfaces. By activating an air suction fan (not illustrated) of the rotary drum 4, an airflow for carrying the material of the absorbent member 104 toward the outer circumferential surface 4f of the rotary drum 4 is created inside the space surrounded by the top plate 31, the bottom plate 32, and the side walls 33, 34 of the duct 3. Stated differently, the inside of the duct 3 serves as a flow path 30.

As illustrated in Fig. 6, the top plate 31 of the duct 3 is provided with an absorbent particle dispersing tube 36 that supplies the absorbent particles 10c into the duct 3. The absorbent particle dispersing tube 36 is configured such that the absorbent particles 10c are discharged, by a device such as a screw feeder (not illustrated), from a dispersing opening provided at the tip end of the absorbent particle dispersing tube 36, and are supplied to inside the duct 3. Further, the supply amount of the absorbent particles 10c to the absorbent particle dispersing tube 36 can be adjusted by the device such as a screw feeder.

As illustrated in Fig. 6, the supplying portion 5 includes: a first cutter roller 53 including a plurality of cutter blades 51 that cut in the first direction (X direction); a second cutter roller 54 including a plurality of cutter blades 52 that cut in the second direction (Y direction); and a single receiving roller 55 arranged in opposition to the first cutter roller 53 and the second cutter roller 54. Also, downstream of the cutter blades 51, 52 in the transporting direction of the synthetic fiber sheet lObs, the supplying portion 5 includes a suction nozzle 58 that sucks the sheet fragments 10bh formed by using the cutter blades 51, 52.

As illustrated in Figs. 6 and 7, the surface of the first cutter roller 53 is provided with a plurality of cutter blades 51, 51, 51,... extending continuously over the entire outer circumference of the first cutter roller 53 along the circumferential direction of the first cutter roller 53, the cutter blades being lined up in the axial direction (X direction) of the first cutter roller 53. By receiving motive power from a prime mover such as a motor, the first cutter roller 53 rotates in the direction of arrow R3. The interval between the cutter blades 51, 51, 51,... adjacent to one another in the axial direction of the first cutter roller 53 substantially corresponds to the width (length in the shorter direction; length in the X direction) of each sheet fragment 10bh formed by cutting. Strictly speaking, depending on the tension during sheet transportation, the synthetic fiber sheet lObs may be cut in a state where it is shrunken in the width direction X; thus, by releasing this tension, the width of each produced sheet fragment 10bh may become wider than the interval between the cutter blades 51, 51, 51,....

As illustrated in Figs. 6 and 7, the surface of the second cutter roller 54 is provided with a plurality of cutter blades 52, 52, 52,... extending continuously over the entire width of the second cutter roller 54 along the axial direction of the second cutter roller 54, the cutter blades being arranged with intervals therebetween in the circumferential direction of the second cutter roller 54. By receiving motive power from a prime mover such as a motor, the second cutter roller 54 rotates in the direction of arrow R4.

As illustrated in Figs. 6 and 7, the receiving roller 55 is a flat roller having a flat surface. By receiving motive power from a prime mover such as a motor, the receiving roller 55 rotates in the direction of arrow R5.

As illustrated in Figs. 6 and 7, opposing the surface of the receiving roller 55, the supplying portion 5 includes, in order from the upstream side toward the downstream side in the rotating direction (the direction of arrow R5): a free roller 56 that introduces the continuous synthetic fiber sheet lObs between the receiving roller 55 and the first cutter roller 53; the first cutter roller 53 that cuts the continuous synthetic fiber sheet lObs in the first direction; a nip roller 57 that introduces, between the receiving roller 55 and the second cutter roller 54, a plurality of continuous sheet fragments 10bh1 (referred to hereinafter also as continuous sheet fragment strips 10bh1) that have been cut in the first direction and extend in the first direction; and the second cutter roller 54 that cuts the continuous sheet fragment strips 10bh1 in the second direction. The supplying portion 5 also includes a feed roller (not illustrated) that transports the continuous synthetic fiber sheet lObs, and the feed roller introduces the continuous synthetic fiber sheet lObs between the receiving roller 55 and the first cutter roller 53. The feed roller is configured so as to be rotated by a driving device such as a servomotor. From the viewpoint of preventing the synthetic fiber sheet lObs from slipping, the feed roller may be made less slippery by forming, in the surface thereof, grooves extending in the axial direction over the entire circumference, or by subjecting the entire circumference to a coating treatment for increasing friction force. Slipping can be suppressed by sandwiching the sheet between the feed roller and a nip roller.

As illustrated in Figs. 6 and 7, the supplying portion 5 includes a suction nozzle 58 that sucks the sheet fragments 10bh formed by the second cutter roller 54. The suction nozzle 58 has a suction opening 581 that is arranged below the second cutter roller 54-i.e., more toward the downstream side, in the second cutter roller 54's rotating direction (the direction of arrow R4), than the closest point between the second cutter roller 54 and the receiving roller 55. The suction opening 581 of the suction nozzle 58 extends over the entire width of the second cutter roller 54. From the viewpoint of improving the ability to suck the sheet fragments 10bh, it is preferable that the suction opening 581 of the suction nozzle 58 is arranged below the receiving roller 55 and the second cutter roller 54 so as to be in opposition between the receiving roller 55 and the second cutter roller 54. From the viewpoint of further improving the ability to suck the sheet fragments 10bh, it is preferable that the suction opening 581 of the suction nozzle 58 covers the outer surface of the second cutter roller 54 such that, as viewed from the side surface of the receiving roller 55 and the second cutter roller 54, the length of an arc of the suction opening 581 opposing the second cutter roller 54 is longer than the length of an arc of the suction opening 581 opposing the receiving roller 55, as illustrated in Fig. 7.

As illustrated in Fig. 6, the suction nozzle 58 is connected by a supply tube 59 to the top plate 31 side of the duct 3. The sheet fragments 10bh sucked from the suction opening 581 of the suction nozzle 58 are supplied to inside the duct 3 in midstream of the duct 3 through the supply tube 59. The connecting position of the supply tube 59 and the duct 3 is located between the defibrating portion 2 side and the rotary drum 4 side in the duct 3, and is located more toward the downstream side, in the duct 3, than the absorbent particle dispersing tube 36. The connecting position of the supply tube 59 and the duct 3 is, however, not limited thereto, and for example, it may be on the bottom plate 32 side and not the top plate 31 side of the duct 3.

As illustrated in Fig. 6, the rotary drum 4 is cylindrical, and includes: a member 40 forming the outer circumferential surface 4f; and a fixed drum body 42 located more inward than the member 40. By receiving motive power from a motor, for example, the member 40 forming the outer circumferential surface 4f rotates in the direction of arrow R1 about a horizontal axis. The member 40 forming the outer circumferential surface 4f is provided with an accumulating depression 41, which serves as an accumulating portion in which the material of the absorbent member is accumulated and an absorbent core 110 is obtained. The accumulating depression 41 is arranged continuously over the entire circumference in the circumferential direction (2Y direction) of the rotary drum 4. The bottom surface of the accumulating depression 41 is constituted by a porous member that functions as suction holes for sucking the material of the absorbent member 104. The drum body 42 has therein a plurality of spaces which are independent from one another. By driving an air suction fan (not illustrated) connected to the rotary drum 4, the pressure in the respective spaces can be adjusted. In the manufacturing device 200, there are three spaces A to C. The suction force in the region corresponding to the space A can be made stronger or weaker than the suction force in the regions corresponding to the spaces B and C, and the space A is maintained at a negative pressure.

As illustrated in Fig. 6, the press-down belt 7 is arranged adjacent to the position of the duct 3 on the downstream side thereof along the rotary drum 4's outer circumferential surface 4f located at the space B. In the space B, the pressure is set to zero (atmospheric pressure) or to a negative pressure weaker than that of the space A of the rotary drum 4. The press-down belt 7 is an endless, air-permeable or air-impermeable belt, bridges rollers 71 and 72, and rotates so as to follow the rotation of the rotary drum 4. Thanks to the press-down belt 7, the continuous absorbent core strip 110r in the accumulating depression 41 can be retained inside the accumulating depression 41 until the absorbent core is transferred onto a vacuum conveyor 8.

As illustrated in Fig. 6, the covering portion 212 covers the continuous absorbent core strip 110r with the continuous core-wrap sheet 111 while transporting the core-wrap sheet 111, to thereby form a continuous absorbent member strip 104r. The covering portion 212 includes: a vacuum conveyor 8; and fold-back guide plates (not illustrated) arranged above the vacuum conveyor 8. The vacuum conveyor 8 is arranged below the rotary drum 4, and is arranged opposing the outer circumferential surface 4f located in the rotary drum 4's space C in which the pressure is set to zero (atmospheric pressure) or to a slightly positive pressure. The vacuum conveyor 8 includes: an endless air-permeable belt 83 that bridges a drive roller 81 and driven rollers 82, 82; and a vacuum box 84 arranged in a position opposing the outer circumferential surface 4f located at the space C of the rotary drum 4 across the air-permeable belt 83. The continuous core-wrap sheet 111, which is made, for example, of tissue paper or a liquid-permeable nonwoven fabric, is introduced onto the vacuum conveyor 8. The fold-back guide plates are members that fold back, in the width direction, the continuous core-wrap sheet 111's extension portions 111R, 111L which extend along the transporting direction, while the continuous core-wrap sheet is transported by the vacuum conveyor 8.

As illustrated in Fig. 5, the absorbent member cutting portion 213 is arranged downstream of the fold-back guide plates. The absorbent member cutting portion 213 includes: a cutter roller 213a having a plurality of cutter blades on its circumferential surface; and an anvil roller 213b having a flat and smooth circumferential surface for receiving the cutter blades. The interval between cutter blades adjacent to one another in the circumferential direction of the cutter roller 213a corresponds to the length in the transporting direction (the length in the length direction) of each absorbent member 104 formed by cutting.

As illustrated in Fig. 5, the absorbent article forming portion 220 includes, in order from the upstream side toward the downstream side in the transporting direction: a topsheet supplying portion 221 that supplies the topsheet 102 onto one surface side of each absorbent member 104; a compressing portion 222 that compresses the absorbent member 104 from above the topsheet 102; a backsheet supplying portion 223 that supplies the backsheet 103 onto the other surface side of each absorbent member 104; a sealing portion 224 that seals the continuous napkin strip 101r into the product shape of each napkin 101; and a napkin cutting portion 225 that cuts the continuous napkin strip 101r to form separate napkins 101.

The topsheet supplying portion 221 includes an introduction roller 221f that introduces a continuous topsheet 102, which is supplied from an original textile roll 2f, onto one surface side of the absorbent members 104 being transported. The backsheet supplying portion 223 includes an introduction roller 223f that introduces a continuous backsheet 103, which is supplied from an original textile roll 3f, onto the other surface side of the absorbent members 104 being transported.

The compressing portion 222 includes an embossing roller 222a having, on the roller surface, a projection corresponding to the depression 108 to be formed in the topsheet 102 and the absorbent member 104; and an anvil roller 222b arranged in opposition to the embossing roller 222a. For the embossing roller 222a, it is possible to use, without particular limitation, a known embossing roller for compressing an absorbent article such as a sanitary napkin.

The sealing portion 224 includes: a pressurizing roller 224a having, on the roller surface, a projection corresponding to the outer shape of each napkin 101; and an anvil roller 224b arranged in opposition to the pressurizing roller 224a. For the pressurizing roller 224a, it is possible to use, without particular limitation, a known pressurizing roller for performing sealing in a shape corresponding to the outer shape of an absorbent article such as a sanitary napkin.

The napkin cutting portion 225 includes: a cutter roller 225a having a cutter blade corresponding to the outer shape of each napkin 101; and an anvil roller 225b arranged in opposition to the cutter roller 225a. For the cutter roller 225a, it is possible to use, without particular limitation, a known cutter roller for performing cutting in a shape corresponding to the outer shape of an absorbent article such as a sanitary napkin.

As illustrated in Fig. 5, the individual package forming portion 230 includes, in order from the upstream side toward the downstream side in the transporting direction: a turning portion 231 that turns each napkin 101 by 90 degrees with respect to the transporting direction; a packaging material attachment portion 232 that attaches a packaging material 105 to each napkin 101; a fold-up portion 233 that folds up each napkin 101 to which the packaging material 105 has been attached; a width sealing portion 234 that seals a continuous individual package strip 100r along the width direction X orthogonal to the transporting direction; and an individual package cutting portion 235 that cuts the continuous individual package strip 100r to form separate individual packages 100.

As illustrated in Fig. 5, the turning portion 231 includes: an introduction roller 231a that introduces the napkins 101; and a turner 231b that turns each napkin 101 by 90 degrees with respect to the transporting direction. The introduction roller 231a is arranged in opposition to the turner 231b. The introduction roller 231a is formed so that it can hold napkins 101 being transported. The turner 231b has, on its circumferential surface, a plurality of suction heads (not illustrated), each sucking and receiving the napkin 101 which is being held by the introduction roller 231a. The suction head is configured so as to be able to turn the sucked-on napkin 101 by 90 degrees about an axis perpendicular to the suction surface of the suction head, in conjunction with the rotation of the turner 231b.

As illustrated in Fig. 5, the packaging material attachment portion 232 includes: a receiving roller 232a that receives each napkin 101 from the turner 231b; an application portion 232b that applies an adhesive to a continuous packaging material 105; and an attachment roller 232c that attaches each napkin 101 to the continuous packaging material 105. The receiving roller 232a has, on its circumferential surface, a plurality of suction heads (not illustrated), each receiving and sucking the napkin 101 which is being transported by the turner 231b. For the application portion 232b, it is possible to use, for example, a die coater or an application roller. The attachment roller 232c is arranged in opposition to the receiving roller 232a in a state where the continuous packaging material 105, to which the napkins 101 have been attached, is interposed therebetween.

As illustrated in Fig. 5, the fold-up portion 233 includes: a first folding portion 233A that folds up one side, along the transporting direction, of the packaging material 105 to which the napkins 101 have been attached; and a second folding portion 233B that folds up the other side. The first folding portion 233A includes: a first fold guide (not illustrated); and a folding roller 233a for applying a starting point of the first folded/bent portion IP1. The second folding portion 233B includes: a second fold guide (not illustrated); and a folding roller 233b for applying a starting point of the second folded/bent portion IP2.

The width sealing portion 234 includes: a pressurizing roller 234a that intermittently pressurizes, in the width direction X, the continuous individual package strip 100r that has been folded up; and an anvil roller 234b arranged in opposition to the pressurizing roller 234a. For the pressurizing roller 234a, it is possible to use, without particular limitation, a known pressurizing roller for compressing an absorbent article such as a sanitary napkin.

The individual package cutting portion 235 includes: a cutter roller 235a having a plurality of cutter blades on its circumferential surface; and an anvil roller 235b arranged in opposition to the cutter roller 235a. Each cutter blade of the cutter roller 235a is formed along the axial direction of the cutter roller 235a so as to extend continuously over the entire width of the cutter roller 235a. The cutter blades are arranged with intervals therebetween in the circumferential direction of the cutter roller 235a. For the cutter roller 235a, it is possible to use, without particular limitation, a known cutter roller for processing an absorbent article such as a sanitary napkin.

Next, a method for manufacturing an individual package 100 of a napkin 101 by using the aforementioned manufacturing device 200-i.e., an embodiment of the manufacturing method of the present invention-will be described. As illustrated in Fig. 5, the method for manufacturing an individual package 100 of a napkin 101 involves: a core forming step of forming an absorbent core 110 by accumulating a plurality of sheet fragments 10bh including synthetic fibers 10b; an article forming step of first forming a continuous strip 101r of a napkin, as an absorbent article, by superposing, on one another, the absorbent core 110 and a continuous liquid-permeable topsheet 102 that is being transported, and then cutting the continuous napkin strip 101r, to form a napkin 101 as an absorbent article; and a fold-up step of folding the napkin 101, with the topsheet 102 on the inside, so as to form folded/bent portions IP that extend in the width direction y1 of the napkin 101. More preferably, the method for manufacturing an individual package 100 of a napkin 101 according to this embodiment involves: a defibrating step of defibrating a continuous hydrophilic sheet 10as by using the defibrating machine 21 and obtaining hydrophilic fibers 10a; a cutting step of cutting a continuous synthetic fiber sheet lObs, including synthetic fibers 10b, at predetermined lengths in the first direction and the second direction, and forming sheet fragments 10bh; a suction step of sucking the sheet fragments 10bh obtained in the cutting step and supplying them to inside the duct 3; a transporting step of transporting the plurality of sheet fragments 10bh and the hydrophilic fibers 10a to the accumulating depression 41, serving as an accumulating portion, by using the duct 3 serving as a transporting portion; and the aforementioned core forming step of accumulating, in the accumulating depression 41 serving as the accumulating portion, the plurality of sheet fragments 10bh and the hydrophilic fibers 10a transported in the transporting step, and forming an absorbent core 110. The method for manufacturing an individual package 100 of a napkin 101 according to this embodiment will be described in detail below.

First, the space A inside the rotary drum 4 and the inside of the vacuum box 84 for the vacuum conveyor 8 are set to a negative pressure by activating air suction fans (not illustrated) respectively connected thereto. By creating a negative pressure inside the space A, an airflow for transporting the material of the absorbent member 104 to the outer circumferential surface 4f of the rotary drum 4 is created inside the duct 3. Further, the defibrating machine 21 and the rotary drum 4 are rotated, the first cutter roller 53, the second cutter roller 54 and the receiving roller 55 are rotated, and the press-down belt 7 and the vacuum conveyor 8 are activated.

Next, as illustrated in Fig. 6, the defibrating step is performed for supplying a continuous hydrophilic sheet 10as to the defibrating machine 21 by using the pair of feed rollers 23, 23, and obtaining hydrophilic fibers 10a by defibrating the sheet. The hydrophilic fibers 10a, which are a defibrated fiber material, are supplied from the defibrating machine 21 to the duct 3. The pair of feed rollers 23, 23 controls the speed for supplying the hydrophilic sheet 10as to the defibrating machine 21; in the defibrating step, the supplying of the hydrophilic sheet 10as to the defibrating machine 21 is controlled.

Separately from the defibrating step, as illustrated in Fig. 6, the cutting step is performed, wherein the continuous synthetic fiber sheet lObs is cut and the sheet fragments 10bh are formed by using: the first cutter roller 53 including the cutter blades 51 that cut in the first direction; and the second cutter roller 54 including the cutter blades 52 that cut in the second direction. In the cutting step, the first cutter roller 53 which cuts the continuous synthetic fiber sheet lObs in the first direction, the second cutter roller 54 which cuts the sheet in the second direction, and the single receiving roller 55 are used. In the cutting step, the continuous synthetic fiber sheet lObs is introduced between the first cutter roller 53 and the receiving roller 55 and is cut in the first direction to form a plurality of continuous sheet fragment strips 10bh1. Then, the formed continuous sheet fragment strips 10bh1 are transported by the receiving roller 55 and are cut in the second direction between the second cutter roller 54 and the receiving roller 55 to form the sheet fragments 10bh. The cutting step is described in detail below.

In the cutting step, the continuous synthetic fiber sheet lObs is transported by using the aforementioned feed roller (not illustrated). The feed roller controls the speed for transporting the continuous synthetic fiber sheet lObs; in the cutting step, the transportation speed of the continuous synthetic fiber sheet lObs is controlled.

As illustrated in Fig. 7, in the cutting step, the continuous synthetic fiber sheet lObs transported by the feed roller is introduced, by the free roller 56, between the receiving roller 55, which rotates in the direction of arrow R5, and the first cutter roller 53, which rotates in the direction of arrow R3, and, with the plurality of cutter blades 51, 51, 51,..., the continuous synthetic fiber sheet lObs is cut in the first direction at positions with intervals therebetween in the second direction. Performing cutting as described above forms a plurality of continuous sheet fragment strips 10bh1 which extend in the first direction and are arranged side by side in the second direction. The plurality of continuous sheet fragment strips 10bh1 have the same width (length in the second direction). From the viewpoint of ensuring that the sheet fragments 10bh have the necessary dimensions to achieve predetermined effects, it is preferable that the average width of the continuous sheet fragment strips 10bh1 formed in the cutting step is from 0.1 to 10 mm, more preferably from 0.3 to 6 mm, even more preferably from 0.5 to 5 mm. In the present embodiment, the width of each continuous sheet fragment strip 10bh1 cut by the first cutter roller 53 matches the length of the side, in the shorter direction, of each sheet fragment 10bh ultimately formed. Cutting, however, may be performed such that the width of each continuous sheet fragment strip 10bh1 cut by the first cutter roller 53 corresponds to the length of the side, in the length direction, of each sheet fragment 10bh ultimately formed. In this case, the average width of the continuous sheet fragment strips 10bh1 cut by the first cutter roller 53 is preferably from 0.3 to 30 mm, more preferably from 1 to 15 mm, even more preferably from 2 to 10 mm. The plurality of continuous sheet fragment strips 10bh1 that have been formed are transported on the circumferential surface of the receiving roller 55 which rotates in the direction of arrow R5, are transported between the receiving roller 55 and the nip roller 57, and are then introduced between the receiving roller 55 and the second cutter roller 54 by the nip roller 57.

Next, as illustrated in Fig. 7, in the cutting step, the plurality of continuous sheet fragment strips 10bh1, which are arranged side by side in the second direction and extend in the first direction, are introduced between the receiving roller 55, which rotates in the direction of arrow R5, and the second cutter roller 54, which rotates in the direction of arrow R4. Then, with the plurality of cutter blades 52, 52, 52,..., the plurality of continuous sheet fragment strips 10bh1 are cut along the second direction and intermittently in the first direction. Performing cutting as described above forms a plurality of rectangular sheet fragments 10bh in which the length in the first direction is longer than the length in the second direction. The plurality of rectangular sheet fragments 10bh have the same length in the first direction. From the viewpoint of ensuring that the sheet fragments 10bh have the necessary dimensions to achieve predetermined effects, it is preferable that the average length of each sheet fragment 10bh formed in the cutting step is from 0.3 to 30 mm, more preferably from 1 to 15 mm, even more preferably from 2 to 10 mm. In the present embodiment, the length of each sheet fragment 10bh cut by the second cutter roller 54 matches the length of the side, in the length direction, of each sheet fragment 10bh. Cutting, however, may be performed such that the length of each sheet fragment 10bh cut by the second cutter roller 54 corresponds to the length of the side, in the shorter direction, of each sheet fragment 10bh. In this case, the length (width) of each sheet fragment 10bh cut by the second cutter roller 54 is preferably from 0.1 to 10 mm, more preferably from 0.3 to 6 mm, even more preferably from 0.5 to 5 mm.

In the cutting step, the continuous synthetic fiber sheet lObs is cut in the first direction and also cut at a predetermined length in the second direction to thereby obtain the sheet fragments 10bh. Thus, the size of the obtained sheet fragments 10bh can easily be adjusted to an intended size, and a large amount of sheet fragments 10bh with the same size can easily be manufactured with high precision. As described above, since the sheet fragments 10bh with an intended size can be formed with high precision, it is possible to efficiently and continuously manufacture absorbent members having an intended absorbency.

Next, the suction step is performed for sucking the sheet fragments 10bh cut and obtained by cutter rollers 53, 54 and supplying them to inside the duct 3 by using the suction nozzle 58 whose suction opening 581 is arranged below the second cutter roller 54. By arranging the suction opening 581 of the suction nozzle 58 below the second cutter roller 54-i.e., more toward the downstream side, in the second cutter roller 54's rotating direction (the direction of arrow R4), than the closest point between the second cutter roller 54 and the receiving roller 55-the plurality of sheet fragments 10bh cut and formed by the second cutter roller 54 and the receiving roller 55 can be sucked efficiently.

Next, the transporting step is performed for transporting the sucked-in sheet fragments 10bh to the accumulating depression 41 in the outer circumferential surface 4f of the rotary drum 4 by being carried on an airflow. In the transporting step, the plurality of sheet fragments 10bh, having undergone the cutting step and the suction step, are supplied to inside the duct 3 from the top plate 31 side of the duct 3 at a position in midstream of the transporting direction Y of the duct 3, and the supplied sheet fragments 10bh are transported to the accumulating depression 41 of the rotary drum 4 by being carried on an airflow.

In the transporting step, first, the hydrophilic fibers 10a obtained in the defibrating step are supplied to inside the duct 3, and then the plurality of sheet fragments 10bh sucked in the suction step are supplied to inside the duct 3 in midstream of the duct 3. Thus, the sheet fragments 10bh are transported on an airflow in midstream of transporting the hydrophilic fibers 10a to the accumulating depression 41 in a dispersed and airborne state on the airflow. Thus, the sheet fragments 10bh and the hydrophilic fibers 10a are mixed while the sheet fragments 10bh and the hydrophilic fibers 10a are being transported in a dispersed and airborne state on an airflow.

Further, in the transporting step, the absorbent particles 10c are supplied by using the absorbent particle dispersing tube 36, and the sheet fragments 10bh and the absorbent particles 10c are mixed while the sheet fragments 10bh obtained in the cutting step and the absorbent particles 10c are being transported to the accumulating depression 41 on an airflow. In the transporting step, the position of the absorbent particle dispersing tube 36 is located more upstream than the connecting position between the supply tube 59 and the duct 3. Thus, the sheet fragments 10bh, the hydrophilic fibers 10a, and the absorbent particles 10c are mixed together while the absorbent particles 10c are being transported to the accumulating depression 41 in a dispersed and airborne state on an airflow.

Next, the core forming step is performed for accumulating the sheet fragments 10bh, which have been transported in the transporting step, as well as the hydrophilic fibers 10a and the absorbent particles 10c, in the accumulating depression 41 provided in the rotary drum 4's outer circumferential surface 4f, to form an absorbent core 110. The sheet fragments 10bh are introduced from the top plate 31 side of the duct 3 in midstream of transporting the hydrophilic fibers 10a from the upstream side of the duct 3 in the transporting direction. Thus, the hydrophilic fibers 10a that are being transported on the side closer to the bottom plate 32 of the duct 3 are less likely to get mixed with the sheet fragments 10bh. On the other hand, the hydrophilic fibers 10a that are being transported on the side closer to the top plate 31 of the duct 3 are more likely to be mixed together with the sheet fragments 10bh introduced from the top plate 31 side of the duct 3. With this core forming step, an absorbent core 110 is formed, including: a first layer 110t in which the sheet fragments 10bh and the hydrophilic fibers 10a-which are transported on the side closer to the top plate 31 of the duct 3-are mixed; and a second layer 110b that includes the hydrophilic fibers 10a transported on the side closer to the bottom plate 32 of the duct 3 and in which the density of presence of the sheet fragments 10bh is smaller than in the first layer 110t. The first layer 110t is formed on the outer side, in the thickness direction, of the accumulating depression 41, whereas the second layer 110b is formed on the inner side, in the thickness direction, of the accumulating depression 41. In this way, the sheet fragments 10bh and the hydrophilic fibers 10a are accumulated in a manner that the density of presence of the sheet fragment 10bh is varied in the thickness direction of the absorbent core 110.

In the core forming step, the density of presence of the sheet fragments 10bh and the hydrophilic fibers 10a in the thickness direction of the absorbent core 110 can be changed, for example, by changing the position for introducing the sheet fragments 10bh in midstream of the transporting direction of the duct 3 toward the upstream side or the downstream side. Alternatively, the density of presence of the sheet fragments 10bh and the hydrophilic fibers 10a in the thickness direction of the absorbent core 110 can be changed, for example, by setting the position for connecting the supply tube 59 to the duct 3 either on the top plate 31 side or the bottom plate 32 side. For example, the sheet fragments 10bh and the hydrophilic fibers 10a will be mixed more uniformly in the thickness direction of the absorbent core 110 if the position for connecting the supply tube 59, which is for introducing the sheet fragments 10bh in midstream of the transporting direction of the duct 3, is on the top plate 31 side and located more toward the upstream side.

As described above, an absorbent core 110 having a two-layer structure is formed in the accumulating depression 41 of the rotary drum 4, the absorbent core being accumulated so as to include, in the thickness direction: a first layer 110t (see Fig. 4) in which the sheet fragments 10bh and the hydrophilic fibers 10a are mixed; and a second layer 110b (see Fig. 4) in which the density of presence of the sheet fragments 10bh is smaller than in the first layer 110t. The absorbent core 110 formed in the accumulating depression 41 is manufactured continuously over the entire circumference, in the circumferential direction (2Y direction), of the rotary drum 4. After forming this continuous absorbent core strip 110r in which the hydrophilic fibers 10a, the synthetic fibers 10b, and the absorbent particles 10c have accumulated within the accumulating depression 41, the rotary drum 4 is further rotated, and, while pressing down the continuous absorbent core strip 110r in the accumulating depression 41 by the press-down belt 7 which is arranged on the outer circumferential surface 4f located at the space B of the rotary drum 4, the absorbent core is transported to above the vacuum conveyor 8, as illustrated in Fig. 6.

Then, when the continuous absorbent core strip 110r within the accumulating depression 41 reaches a position opposing the vacuum box 84 located at the space C of the rotary drum 4, it is released from the accumulating depression 41 by suction from the vacuum box 84, as illustrated in Fig. 6. Then, the continuous absorbent core strip 110r, which has been released from the accumulating depression 41, is transferred onto a central section on one surface of the core-wrap sheet 111, which is being transported by the vacuum conveyor 8. The continuous absorbent core strip 110r, which has been placed on the one surface of the core-wrap sheet 111, is in a state where the first layer 110t is located on the core-wrap sheet 111 side and the second layer 110b is located on the opposite side from the core-wrap sheet 111 in the thickness direction.

Then, for example, on one surface of the vacuum conveyor 8, one extension portion 111R of the core-wrap sheet 111 is folded back by using a fold-back guide plate (not illustrated) of the covering portion 212 so that the extension portion contacts the surface of the second layer 110b and thereby covers one lateral side edge 110R of the continuous absorbent core strip 100r, as illustrated in Fig. 6. Further, the other extension portion 111L of the core-wrap sheet 111 is folded back so that the extension portion contacts the surface of the second layer 110b and thereby covers the other lateral side edge 110L of the continuous absorbent core strip 100r. In this way, the covering step is performed for manufacturing a continuous absorbent member strip 104r made by folding back the core-wrap sheet 111's both lateral side edges 110R, 110L, which extend along the transporting direction, and covering the entire periphery of the absorbent core 110 by superposing the folded-back lateral sides bR, bL on one another on the surface of the second layer 110b of the absorbent core 110.

Next, by using the pressing portion 214, the continuous absorbent member strip 104r is compressed in the thickness direction Z. Then, as illustrated in Fig. 5, the continuous absorbent member strip 104r is transported to between the cutter roller 213a and the anvil roller 213b of the absorbent member cutting portion 213. The continuous absorbent member strip 104r is then cut at predetermined intervals in the transporting direction, to thereby form separate absorbent members 104. Since sheet fragments 10bh including synthetic fibers with an intended size are dispersed within the absorbent member 104, by using a napkin 101 including the thus-manufactured absorbent member 104, there is less likelihood that uncomfortableness of contacting a foreign object is felt during use, and also, body fluid can be absorbed stably when absorbing body fluid with the absorbent member 104.

Next, the article forming step is performed, wherein, first, a continuous napkin strip 101r is formed by using the separate absorbent members 104, and then the continuous napkin strip 101r is cut to manufacture separate napkins 101. As illustrated in Fig. 5, in the article forming step, a continuous topsheet 102, which is supplied from an original textile roll 2f, is introduced onto one surface side of each absorbent member 104 by the introduction roller 221f, and the absorbent member 104 (i.e., the absorbent core 110 and the core-wrap sheet 111) and the continuous topsheet 102 are superposed on one another such that the first layer 110t of the absorbent core 110 is arranged on the topsheet 102 side.

Next, as illustrated in Fig. 5, the continuous topsheet 102, on which the absorbent members 104 have been superposed, is transported to between the embossing roller 222a and the anvil roller 222b of the compressing portion 222. Then, by using the projection corresponding to the depression 108 to be formed in each absorbent member 104, the absorbent member 104 is compressed from above the topsheet 102, to form the depression 108.

Next, as illustrated in Fig. 5, a continuous backsheet 103 supplied from an original textile roll 3f is introduced by the introduction roller 223f, and the continuous backsheet 103 is superposed onto the other surface side of each absorbent member 104 having been integrated with the topsheet 102 by the depression 108. Then, the absorbent members 104, which are sandwiched between the topsheet 102 and the backsheet 103, are transported to between the pressurizing roller 224a and the anvil roller 224b of the sealing portion 224. Then, by using the projection corresponding to the outer shape of each napkin 101, each absorbent member 104, which is sandwiched between the topsheet 102 and the backsheet 103, is subjected to joining in a shape corresponding to the product shape, to thereby form a continuous napkin strip 101r.

Next, as illustrated in Fig. 5, the continuous napkin strip 101r is transported to between the cutter roller 225a and the anvil roller 225b of the napkin cutting portion 225. Then, by using the cutter blade corresponding to the outer shape of the napkin 101, the continuous napkin strip 101r is cut along the sealed section, to thereby form separate napkins 101.

Next, as illustrated in Fig. 5, each napkin 101 being transported is flipped over such that the upper surface and the lower surface of each napkin 101 formed in the article forming step are inverted. Then, by using the introduction roller 231a of the turning portion 231, each inverted napkin 101 is transferred to the turner 231b. The turner 231b sucks one surface of the inverted napkin 101 with the suction head (not illustrated), and turns the napkin 101 by 90 degrees with respect to the transporting direction (see Fig. 8). Thus, the length direction of the napkin matches the direction orthogonal to the transporting direction.

As illustrated in Fig. 5, an adhesive is applied, by using the application portion 232b of the packaging material attachment portion 232, to a continuous packaging material 105 supplied from an original textile roll 105f. The adhesive is applied to one surface of the packaging material 105 that comes into opposition to the non-skin-facing surface of the napkin 101. The adhesive, however, may instead be applied to the non-skin-facing surface of each napkin 101. Then, the continuous packaging material 105 is transported to between the receiving roller 232a, which receives the napkins 101 from the turner 231b, and the attachment roller 232c, and, as illustrated in Fig. 8, the napkins 101 are attached intermittently onto the continuous packaging material 105 being transported. Then, the fold-up step is performed for folding each napkin 101, which has been attached to the continuous packaging material 105, together with the packaging material 105, with the topsheet 102 of the napkin 101 on the inside.

In the fold-up step, at the first folding portion 233A of the fold-up portion 233, each napkin 101, which has been attached intermittently to the packaging material 105, is folded at a section where the sheet fragments 10bh are provided, with the topsheet 102 on the inside, together with the packaging material 105's one lateral side extending along the transporting direction, thereby forming the first folded/bent portion IP1. Then, the packaging material 105's one lateral side is folded up together with the napkin 101 while pressing the one lateral side with the folding roller 233a. Similarly, by using the second fold guide (not illustrated), each napkin 101 is folded at a section where the sheet fragments 10bh are provided, with the topsheet 102 on the inside, together with the packaging material 105's other lateral side extending along the transporting direction, thereby forming the second folded/bent portion IP2. Then, the packaging material 105's other lateral side is folded up together with the napkin 101 while pressing the other lateral side with the folding roller 233b. This forms a continuous individual package strip 100r, which includes napkins 101 packaged by the packaging material 105 wherein the napkins 101 are in a folded-up state.

Next, as illustrated in Figs. 5 and 8, the continuous individual package strip 100r, in a state where both lateral sides extending along the transporting direction have been folded up, is transported to between the pressurizing roller 234a and the anvil roller 234b of the width sealing portion 234, and the continuous individual package strip 100r is sealed along the width direction orthogonal to the transporting direction. The location for sealing the continuous individual package strip 100r along the width direction is between adjacent napkins 101, 101 which have been attached to the packaging material 105. Stated differently, sealing is performed intermittently in the transporting direction so as to form a sealed region between adjacent napkins 101, 101 of the continuous individual package strip 100r being transported.

Next, as illustrated in Figs. 5 and 8, the continuous individual package strip 100r, in which sealed regions have been formed at the width sealing portion 234, is transported to between the cutter roller 235a and the anvil roller 235b of the individual package cutting portion 235, to cut each sealed region in the continuous individual package strip 100r along the width direction X and thereby form separate individual packages 100.

The aforementioned individual package 100 includes folded/bent portions IP formed by folding, in the fold-up step, the napkin 101, which includes the absorbent core 110 including the sheet fragments 10bh, at a section of the absorbent core 110 where the sheet fragments 10bh are provided. Since the sheet fragments 10bh, which have recoverability that allows easy recovery to the original state, are arranged on the topsheet 102 side of the absorbent core 110, it is possible to manufacture individual packages 100 in which folding creases are less likely to be formed in the surface of the topsheet 102 at positions corresponding to the folded/bent portions IP of the napkin 101 when the napkin 101 is spread open from its individually-packaged state.

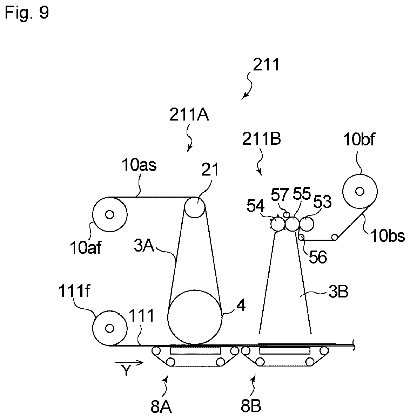

Next, another embodiment of a method for manufacturing the aforementioned individual package 100 of a napkin 101 is described with reference to Fig. 9. Fig. 9 schematically illustrates a portion of another embodiment of the manufacturing device 200 illustrated in Fig. 5. Below, features that are different from those of the manufacturing device illustrated in Fig. 5 will be described. Features that are not particularly explained are the same as those in the manufacturing device 200 illustrated in Fig. 5; features that are the same are accompanied by the same reference signs employed in the description of the manufacturing device 200 illustrated in Fig. 5 and the manufacturing method employing the manufacturing device 200, and explanation thereon is omitted.

In the manufacturing method employing the manufacturing device 200 illustrated in Fig. 5, the absorbent core 110 is formed by supplying the sheet fragments 10bh and the hydrophilic fibers 10a to inside a single duct 3. In the manufacturing method employing the manufacturing device 200 illustrated in Fig. 9, an absorbent core 110 is manufactured by separately forming hydrophilic fibers 10a and sheet fragments 10bh by using separate ducts 3A, 3B.

The core forming portion 211 illustrated in Fig. 9 includes: a second layer forming portion 211A that forms the second layer 110b illustrated in Fig. 4 in which no sheet fragment 10bh is present; and a first layer forming portion 211B that forms the first layer 110t illustrated in Fig. 4 including the sheet fragments 10bh. As illustrated in Fig. 9, the second layer forming portion 211A includes: a defibrating machine 21 that defibrates a hydrophilic sheet 10as; a first duct 3A that transports hydrophilic fibers 10a on an airflow; a rotary drum 4 in which the hydrophilic fibers 10a are accumulated; and a first vacuum conveyor 8A arranged below the rotary drum 4. As illustrated in Fig. 9, the first layer forming portion 211B includes: a first cutter roller 53; a second cutter roller 54; a single receiving roller 55; a second duct 3B that transports sheet fragments 10bh; and a second vacuum conveyor 8B arranged below the second duct 3B.

The first duct 3A is configured similarly to the duct 3, except that the supply tube 59 for supplying the sheet fragments 10bh is not connected to the top plate 31 of the duct 3. The upstream-side opening of the second duct 3B is arranged on the downstream side of the second cutter roller 54 and the receiving roller 55, and extends over the entire width of the second cutter roller 54. The first vacuum conveyor 8A and the second vacuum conveyor 8B have the same configuration as the vacuum conveyor 8.

First, as illustrated in Fig. 9, a continuous hydrophilic sheet 10as supplied from an original textile roll lOaf is supplied to and defibrated by the defibrating machine 21, to obtain hydrophilic fibers 10a. Then, by setting the inside of the vacuum box 84 of the first vacuum conveyor 8A to a negative pressure, an airflow that flows inside the first duct 3A is created. Then, the hydrophilic fibers 10a are accumulated on the core-wrap sheet 111 by employing the created airflow, to thereby form the second layer 110b illustrated in Fig. 4 that consists only of the hydrophilic fibers 10a and in which no sheet fragment 10bh is present.

Next, as illustrated in Fig. 9, a continuous synthetic fiber sheet lObs supplied from an original textile roll 10bf is cut by using the first cutter roller 53 and the second cutter roller 54, to form sheet fragments 10bh. By setting the inside of the vacuum box 84 of the second vacuum conveyor 8B to a negative pressure, an airflow that flows inside the second duct 3B is created. Then, the sheet fragments 10bh that have been cut in the cutting step are accumulated on the second layer 110b being transported on the core-wrap sheet 111 by employing the airflow flowing inside the second duct 3B. In this way, the absorbent core 110 illustrated in Fig. 4-which includes the first layer 110t including the sheet fragments 10bh and the second layer not including the sheet fragments 10bh-is manufactured. By using the manufacturing device 200 illustrated in Fig. 9, it is possible to reliably form, in the core forming step, an absorbent core 110 in which the second layer 110b does not include the sheet fragments 10bh, because the second layer forming portion 211A is arranged upstream of the first layer forming portion 211B. After forming the absorbent core 110, the topsheet 102 and the absorbent core 110 are superposed on one another in the article forming step in a manner that the first layer 110t of the absorbent core 110 is arranged on the topsheet 102 side. According to the individual package 100 of the napkin 101 including the thus-formed absorbent core 110, the density of presence of the sheet fragments 10bh on the topsheet 102 side of the absorbent core 110 can be increased efficiently, and thus, individual packages 100 in which folding creases are less likely to be formed can be manufactured efficiently.

The present invention is not limited to the foregoing embodiments and can be modified as appropriate.

For example, in the aforementioned individual package 100, the absorbent core 110 includes the sheet fragments 10bh, the hydrophilic fibers 10a, and the absorbent particles 10c, but the absorbent core may be made only of the sheet fragments 10bh, and the sheet fragments 10bh may be entangled and joined with one another. Alternatively, the first layer 110t may be formed of the sheet fragments 10bh and the absorbent particles 10c without including the hydrophilic fibers 10a, and the second layer 110b may be formed by including the sheet fragments 10bh, the hydrophilic fibers 10a, and the absorbent particles 10c. Alternatively, the absorbent core 110 may be formed of the sheet fragments 10bh and the hydrophilic fibers 10a, without including the absorbent particles 10c.

In the aforementioned individual package 100, the absorbent core 110 includes the first layer 110t and the second layer 110b, and the sheet fragments 10bh are present in the first layer 110t. In cases where the absorbent core 110 is a laminate including three or more layers, it is preferable that the sheet fragments 10bh are present in the layer located closest to the topsheet 102 side.

Further, in the aforementioned individual package 100, the sheet fragments 10bh are not present in the second layer 110b of the absorbent core 110. It will suffice, however, if the density of presence of the sheet fragments 10bh in the second layer 110b of the absorbent core 110 is smaller than the density of presence of the sheet fragments 10bh in the first layer.

Further, in the foregoing embodiments, the sheet fragments 10bh are manufactured by performing the cutting step. Instead, sheet fragments 10bh manufactured in advance may be used. Also, sheet fragments 10bh manufactured according to methods other than by cutter blades may be used. Further, in the cutting step of the present embodiment, sheet fragments 10bh having the same size are manufactured by cutting the synthetic fiber sheet lObs by using the first cutter roller 53 having a plurality of cutter blades 51 arranged at even intervals and the second cutter roller 54 having a plurality of cutter blades 52 arranged at even intervals, as illustrated in Fig. 7. However, the cutting direction of the sheet fragments 10bh and the shape of the sheet fragments 10bh are not limited, and for example, the sheet fragments 10bh may be manufactured by cutting the synthetic fiber sheet lObs by using a first cutter roller 53 having a plurality of cutter blades 51 arranged at two or more types of intervals, or a second cutter roller 54 having a plurality of cutter blades 52 arranged at two or more types of intervals. Manufacturing in this way can form sheet fragments 10bh having two or more sizes, but unlike manufacturing by employing a cutter mill system, sheet fragments with intended sizes can be formed with high precision, and absorbent members having an intended absorbency can be manufactured efficiently and continuously.

In the manufacturing device 200 illustrated in Fig. 6, the supplying portion 5 includes the first cutter roller 53 and the second cutter roller 54, but instead of the two cutter rollers, the supplying portion may include a single cutter roller having, on the same circumferential surface, cutter blades 51 that cut in the first direction (Y direction) and cutter blades 52 that cut in the second direction (X direction). In cases where the supplying portion 5 includes the aforementioned single cutter roller, it is preferable that the supplying portion includes a single receiving roller arranged in opposition to the single cutter roller. In a manufacturing device including the aforementioned single cutter roller and single receiving roller, it is preferable that the suction opening 581 of the suction nozzle 58 is arranged below the single cutter roller.

Further, as illustrated in Fig. 7, in the cutting step of the present embodiment, the sheet fragments 10bh are manufactured by cutting the synthetic fiber sheet lObs by using the first cutter roller 53 and the second cutter roller 54, but instead of using cutter rollers, the sheet fragments 10bh may be manufactured by cutting the synthetic fiber sheet lObs by using a press machine including cutter blades 51 that cut in the first direction (Y direction) and a press machine including cutter blades 52 that cut in the second direction (X direction).

The shape of the absorbent core 110 to be manufactured may be changed flexibly by changing the shape of the accumulating depression 41. Further, the fibers used for the synthetic fibers 10b may be subjected to a hydrophilizing treatment.

In relation to the foregoing embodiments, the following absorbent member manufacturing methods are further disclosed.
{1} An absorbent article package comprising an absorbent article including a topsheet, a backsheet, and an absorbent core arranged between the topsheet and the backsheet, the absorbent article having a longitudinal direction corresponding to a front-rear direction of a wearer and a lateral direction orthogonal to the longitudinal direction, the absorbent article being packaged in a folded-up state, wherein:
   the absorbent article is folded up, with the topsheet on the inside, along a folded/bent portion that extends in the lateral direction of the absorbent article;
   the absorbent core includes a plurality of sheet fragments including synthetic fibers; and
   the sheet fragments are provided at least on the topsheet side in a thickness direction of the absorbent core.
{2} The absorbent article package as set forth in clause {1}, wherein the absorbent core includes hydrophilic fibers.
{3} The absorbent article package as set forth in clause {2}, wherein the absorbent core includes, in the thickness direction:
   a first layer on the topsheet side and in which the sheet fragments and the hydrophilic fibers are mixed; and
   a second layer on the backsheet side and in which a density of presence of the sheet fragments is smaller than in the first layer.
{4} The absorbent article package as set forth in clause {3}, wherein the sheet fragments are not present in the second layer.
{5} The absorbent article package as set forth in any one of clauses {1} to {4}, wherein the absorbent core is covered by a core-wrap sheet.
{6} The absorbent article package as set forth in any one of clauses {1} to {5}, wherein the absorbent core includes a depression that is depressed from the topsheet side toward the backsheet side.
{7} The absorbent article package as set forth in clause {6}, wherein the depression is formed in a manner that the topsheet and the absorbent core are integrally depressed.
{8} The absorbent article package as set forth in any one of clauses {1} to {7}, wherein the folded/bent portion includes a first folded/bent portion and a second folded/bent portion that are separated from one another in the longitudinal direction of the absorbent article.
{9} The absorbent article package as set forth in any one of clauses {1} to {8}, wherein:
   the absorbent article package includes a packaging material in which the absorbent article is packaged; and
   the packaging material packages the entire absorbent article by being folded up along the folded/bent portion together with the absorbent article.
{10} The absorbent article package as set forth in any one of clauses {1} to {9}, wherein the average length of the sheet fragments is preferably from 0.3 to 30 mm, more preferably from 1 to 15 mm, even more preferably from 2 to 10 mm.
{11} The absorbent article package as set forth in any one of clauses {1} to {10}, wherein the average width of the sheet fragments is preferably from 0.1 to 10 mm, more preferably from 0.5 to 6 mm, even more preferably from 0.5 to 5 mm.
{12} The absorbent article package as set forth in any one of clauses {1} to {11}, wherein the average thickness of the sheet fragments is preferably from 0.001 to 10 mm, more preferably from 0.01 to 5 mm.
{13} The absorbent article package as set forth in any one of clauses {1} to {12}, wherein the content of the sheet fragments in the absorbent core with respect to the entire mass of the absorbent core in a dry state is preferably 1 mass% or greater, more preferably 10 mass% or greater, and preferably 100 mass% or less, more preferably 90 mass% or less, and preferably from 1 to 100 mass%, more preferably from 10 to 90 mass%.
{14} The absorbent article package as set forth in any one of clauses {1} to {13}, wherein:
   the absorbent core includes hydrophilic fibers; and
   the content of the hydrophilic fibers in the absorbent core with respect to the entire mass of the absorbent core in a dry state is preferably 1 mass% or greater, more preferably 10 mass% or greater, and preferably 99 mass% or less, more preferably 90 mass% or less, and preferably from 1 to 99 mass%, more preferably from 10 to 90 mass%.
{15} The absorbent article package as set forth in any one of clauses {1} to {14}, wherein the basis weight of the sheet fragments in the absorbent core is preferably 1 g/m² or greater, more preferably 20 g/m² or greater, and preferably 1000 g/m² or less, more preferably 800 g/m² or less, and preferably from 1 to 1000 g/m², more preferably from 20 to 800 g/m².
{16} The absorbent article package as set forth in any one of clauses {1} to {15}, wherein:
   the absorbent core includes hydrophilic fibers; and
   the basis weight of the hydrophilic fibers in the absorbent core is preferably 1 g/m² or greater, more preferably 20 g/m² or greater, and preferably 1000 g/m² or less, more preferably 800 g/m² or less, and preferably from 1 to 1000 g/m², more preferably from 20 to 800 g/m².
{17} The absorbent article package as set forth in any one of clauses {1} to {16}, wherein:
   the absorbent core includes absorbent particles; and
   the content of the absorbent particles in the absorbent core with respect to the entire mass of the absorbent core in a dry state is preferably 0 mass% or greater, more preferably 1 mass% or greater, and preferably 90 mass% or less, more preferably 70 mass% or less, and preferably from 0 to 90 mass%, more preferably from 1 to 70 mass%.
{18} The absorbent article package as set forth in any one of clauses {1} to {17}, wherein:
   the absorbent core includes absorbent particles; and
   the basis weight of the absorbent particles in the absorbent core is preferably 0 g/m² or greater, more preferably 5 g/m² or greater, and preferably 1000 g/m² or less, more preferably 800 g/m² or less, and preferably from 0 to 1000 g/m², more preferably from 5 to 800 g/m².
{19} The absorbent article package as set forth in any one of clauses {1} to {18}, wherein:
   the absorbent core includes hydrophilic fibers;
   the absorbent core includes, in the thickness direction:
      a first layer on the topsheet side and in which the sheet fragments and the hydrophilic fibers are mixed; and
      a second layer on the backsheet side and in which the density of presence of the sheet fragments is smaller than in the first layer; and
   the density of presence of the sheet fragments in the first layer of the absorbent core is preferably 1 piece/cm² or greater, more preferably 5 pieces/cm² or greater, and preferably 500 pieces/cm² or less, more preferably 200 pieces/cm² or less, and preferably from 1 to 500 pieces/cm², more preferably from 5 to 200 pieces/cm².
{20} The absorbent article package as set forth in any one of clauses {1} to {19}, wherein:
   the absorbent core includes hydrophilic fibers;
   the absorbent core includes, in the thickness direction:
      a first layer on the topsheet side and in which the sheet fragments and the hydrophilic fibers are mixed; and
      a second layer on the backsheet side and in which the density of presence of the sheet fragments is smaller than in the first layer; and
   the density of presence of the sheet fragments in the second layer of the absorbent core is preferably 0 pieces/cm² or greater, more preferably 1 piece/cm² or greater, and preferably 500 pieces/cm² or less, more preferably 200 pieces/cm² or less, and preferably from 0 to 500 pieces/cm², more preferably from 20 to 200 pieces/cm².
{21} A method for manufacturing an absorbent article package that includes an absorbent article including a topsheet, a backsheet, and an absorbent core arranged between the topsheet and the backsheet, the absorbent article having a longitudinal direction corresponding to a front-rear direction of a wearer and a lateral direction orthogonal to the longitudinal direction, the absorbent article being packaged in a folded-up state, the method comprising:
   a core forming step of forming the absorbent core by accumulating a plurality of sheet fragments including synthetic fibers;
   an article forming step of
      first forming a continuous absorbent article strip by superposing, on one another, the absorbent core and a continuous topsheet that is being transported, and
      then cutting the continuous absorbent article strip, to form the absorbent article; and
   a fold-up step of folding the absorbent article, with the topsheet on the inside, so as to form a folded/bent portion that extends in the lateral direction of the absorbent article, wherein:
      in the fold-up step, the folded/bent portion is formed by performing folding at a section where the sheet fragments are present in the absorbent core.
{22} The method for manufacturing an absorbent article package as set forth in clause {21}, wherein:
   the method comprises a defibrating step of defibrating a continuous hydrophilic sheet and obtaining hydrophilic fibers; and
   in the core forming step, the absorbent core is formed including the sheet fragments and the hydrophilic fibers.
{23} The method for manufacturing an absorbent article package as set forth in clause {22}, wherein:
   in the core forming step, the absorbent core is formed including
      a first layer in which the sheet fragments and the hydrophilic fibers are mixed, and
      a second layer in which a density of presence of the sheet fragments is smaller than in the first layer; and
   in the article forming step, the topsheet and the absorbent core are superposed in a manner that the first layer of the absorbent core is arranged on the topsheet side.
{24} The method for manufacturing an absorbent article package as set forth in clause {23}, wherein, in the core forming step, the second layer is formed in which the sheet fragments are not present.
{25} The method for manufacturing an absorbent article package as set forth in any one of clauses {21} to {24}, wherein, in the article forming step, a depression is formed by compressing the absorbent core from above the topsheet superposed on the absorbent core.
{26} The method for manufacturing an absorbent article package as set forth in any one of clauses {21} to {25}, wherein, in the article forming step, the continuous absorbent article strip is formed by superposing the backsheet on the absorbent core and joining the topsheet and the backsheet together.
{27} The method for manufacturing an absorbent article package as set forth in any one of clauses {21} to {26}, wherein:
   the absorbent articles formed in the article forming step are attached intermittently to a continuous packaging material that is being transported; and
   in the fold-up step, the absorbent article is folded together with the packaging material.
{28} The method for manufacturing an absorbent article package as set forth in any one of clauses {21} to {27}, wherein:
   the method further comprises a cutting step of cutting a continuous synthetic fiber sheet including the synthetic fibers at predetermined lengths in a first direction and a second direction intersecting with the first direction, and forming the plurality of sheet fragments; and
   in the core forming step, the absorbent core is formed by accumulating the plurality of sheet fragments formed in the cutting step.
{29} The method for manufacturing an absorbent article package as set forth in clause {28}, wherein, in the cutting step:
   the continuous synthetic fiber sheet is cut and continuous sheet fragment strips are formed by using a first cutter roller including cutter blades that cut in the first direction; and
   the continuous sheet fragment strips are cut and the plurality of sheet fragments are formed by using a second cutter roller including cutter blades that cut in the second direction.
{30} The method for manufacturing an absorbent article package as set forth in clause {28} or {29}, wherein the first direction is a direction in which the continuous synthetic fiber sheet is transported in the cutting step, and the second direction is a direction orthogonal to the first direction.
{31} The method for manufacturing an absorbent article package as set forth in any one of clauses {28} to {30}, wherein the average length of the sheet fragments formed in the cutting step is preferably from 0.3 to 30 mm, more preferably from 1 to 15 mm, even more preferably from 2 to 10 mm.
{32} The method for manufacturing an absorbent article package as set forth in any one of clauses {28} to {31}, wherein the average width of the sheet fragments formed in the cutting step is preferably from 0.1 to 10 mm, more preferably from 0.3 to 6 mm, even more preferably from 0.5 to 5 mm.
{33} The method for manufacturing an absorbent article package as set forth in any one of clauses {21} to {32}, wherein:
   the absorbent articles formed in the article forming step are attached intermittently to a continuous packaging material that is being transported; and
   in the fold-up step, the absorbent article is folded together with the packaging material.

### Industrial Applicability

The present invention can provide an absorbent article package in which folding creases are less likely to be formed in the topsheet. The present invention can also provide a method for manufacturing an absorbent article package in which folding creases are less likely to be formed in the topsheet.

## Claims

1. An absorbent article package comprising an absorbent article including a topsheet, a backsheet, and an absorbent core arranged between the topsheet and the backsheet, the absorbent article having a longitudinal direction corresponding to a front-rear direction of a wearer and a lateral direction orthogonal to the longitudinal direction, the absorbent article being packaged in a folded-up state, wherein:
the absorbent article is folded up, with the topsheet on the inside, along a folded/bent portion that extends in the lateral direction of the absorbent article;
the absorbent core includes a plurality of sheet fragments including synthetic fibers; and
the sheet fragments are provided at least on the topsheet side in a thickness direction of the absorbent core.

2. The absorbent article package according to claim 1, wherein the absorbent core includes hydrophilic fibers.

3. The absorbent article package according to claim 2, wherein the absorbent core includes, in the thickness direction:
a first layer on the topsheet side and in which the sheet fragments and the hydrophilic fibers are mixed; and
a second layer on the backsheet side and in which a density of presence of the sheet fragments is smaller than in the first layer.

4. The absorbent article package according to claim 3, wherein the sheet fragments are not present in the second layer.

5. The absorbent article package according to any one of claims 1 to 4, wherein the absorbent core is covered by a core-wrap sheet.

6. The absorbent article package according to any one of claims 1 to 5, wherein the absorbent core includes a depression that is depressed from the topsheet side toward the backsheet side.

7. The absorbent article package according to claim 6, wherein the depression is formed in a manner that the topsheet and the absorbent core are integrally depressed.

8. The absorbent article package according to any one of claims 1 to 7, wherein the folded/bent portion includes a first folded/bent portion and a second folded/bent portion that are separated from one another in the longitudinal direction of the absorbent article.

9. The absorbent article package according to any one of claims 1 to 8, wherein:
the absorbent article package includes a packaging material in which the absorbent article is packaged; and
the packaging material packages the entire absorbent article by being folded up along the folded/bent portion together with the absorbent article.

10. The absorbent article package according to any one of claims 1 to 9, wherein an average length of the sheet fragments is from 0.3 to 30 mm.

11. The absorbent article package according to any one of claims 1 to 10, wherein an average width of the sheet fragments is from 0.1 to 10 mm.

12. The absorbent article package according to any one of claims 1 to 11, wherein an average thickness of the sheet fragments is from 0.001 to 10 mm.

13. The absorbent article package according to any one of claims 1 to 12, wherein a content of the sheet fragments in the absorbent core with respect to an entire mass of the absorbent core in a dry state is from 1 to 100 mass%.

14. The absorbent article package according to any one of claims 1 to 13, wherein:
the absorbent core includes hydrophilic fibers; and
a content of the hydrophilic fibers in the absorbent core with respect to the entire mass of the absorbent core in a dry state is from 1 to 99 mass%.

15. The absorbent article package according to any one of claims 1 to 14, wherein a basis weight of the sheet fragments in the absorbent core is from 1 to 1000 g/m².

16. The absorbent article package according to any one of claims 1 to 15, wherein:
the absorbent core includes hydrophilic fibers; and
a basis weight of the hydrophilic fibers in the absorbent core is from 1 to 1000 g/m².

17. The absorbent article package according to any one of claims 1 to 16, wherein:
the absorbent core includes absorbent particles; and
a content of the absorbent particles in the absorbent core with respect to the entire mass of the absorbent core in a dry state is from 0 to 90 mass%.

18. The absorbent article package according to any one of claims 1 to 17, wherein:
the absorbent core includes absorbent particles; and
a basis weight of the absorbent particles in the absorbent core is from 0 to 1000 g/m².

19. The absorbent article package according to any one of claims 1 to 18, wherein:
the absorbent core includes hydrophilic fibers;
the absorbent core includes, in the thickness direction:
a first layer on the topsheet side and in which the sheet fragments and the hydrophilic fibers are mixed; and
a second layer on the backsheet side and in which the density of presence of the sheet fragments is smaller than in the first layer; and
the density of presence of the sheet fragments in the first layer of the absorbent core is from 1 to 500 pieces/cm².

20. The absorbent article package according to any one of claims 1 to 19, wherein:
the absorbent core includes hydrophilic fibers;
the absorbent core includes, in the thickness direction:
a first layer on the topsheet side and in which the sheet fragments and the hydrophilic fibers are mixed; and
a second layer on the backsheet side and in which the density of presence of the sheet fragments is smaller than in the first layer; and
the density of presence of the sheet fragments in the second layer of the absorbent core is from 0 to 500 pieces/cm².

21. A method for manufacturing an absorbent article package that includes an absorbent article including a topsheet, a backsheet, and an absorbent core arranged between the topsheet and the backsheet, the absorbent article having a longitudinal direction corresponding to a front-rear direction of a wearer and a lateral direction orthogonal to the longitudinal direction, the absorbent article being packaged in a folded-up state, the method comprising:
a core forming step of forming the absorbent core by accumulating a plurality of sheet fragments including synthetic fibers;
an article forming step of
first forming a continuous absorbent article strip by superposing, on one another, the absorbent core and a continuous topsheet that is being transported, and
then cutting the continuous absorbent article strip, to form the absorbent article; and
a fold-up step of folding the absorbent article, with the topsheet on the inside, so as to form a folded/bent portion that extends in the lateral direction of the absorbent article, wherein:
in the fold-up step, the folded/bent portion is formed by performing folding at a section where the sheet fragments are present in the absorbent core.

22. The method for manufacturing an absorbent article package according to claim 21, wherein:
the method comprises a defibrating step of defibrating a continuous hydrophilic sheet and obtaining hydrophilic fibers; and
in the core forming step, the absorbent core is formed including the sheet fragments and the hydrophilic fibers.

23. The method for manufacturing an absorbent article package according to claim 22, wherein:
in the core forming step, the absorbent core is formed including
a first layer in which the sheet fragments and the hydrophilic fibers are mixed, and
a second layer in which a density of presence of the sheet fragments is smaller than in the first layer; and
in the article forming step, the topsheet and the absorbent core are superposed in a manner that the first layer of the absorbent core is arranged on the topsheet side.

24. The method for manufacturing an absorbent article package according to claim 23, wherein, in the core forming step, the second layer is formed in which the sheet fragments are not present.

25. The method for manufacturing an absorbent article package according to any one of claims 21 to 24, wherein, in the article forming step, a depression is formed by compressing the absorbent core from above the topsheet superposed on the absorbent core.

26. The method for manufacturing an absorbent article package according to any one of claims 21 to 25, wherein, in the article forming step, the continuous absorbent article strip is formed by superposing the backsheet on the absorbent core and joining the topsheet and the backsheet together.

27. The method for manufacturing an absorbent article package according to any one of claims 21 to 26, wherein:
the method further comprises a cutting step of cutting a continuous synthetic fiber sheet including the synthetic fibers at predetermined lengths in a first direction and a second direction intersecting with the first direction, and forming the plurality of sheet fragments; and
in the core forming step, the absorbent core is formed by accumulating the plurality of sheet fragments formed in the cutting step.

28. The method for manufacturing an absorbent article package according to claim 27, wherein, in the cutting step:
the continuous synthetic fiber sheet is cut and continuous sheet fragment strips are formed by using a first cutter roller including cutter blades that cut in the first direction; and
the continuous sheet fragment strips are cut and the plurality of sheet fragments are formed by using a second cutter roller including cutter blades that cut in the second direction.

29. The method for manufacturing an absorbent article package according to any one of claims 21 to 28, wherein:
the absorbent articles formed in the article forming step are attached intermittently to a continuous packaging material that is being transported; and
in the fold-up step, the absorbent article is folded together with the packaging material.
